(19) Europäisches Patentamt
European Patent Office
Office européen des brevets

(11) Veröffentlichungsnummer: **0 387 762**
**A2**

(12) # EUROPÄISCHE PATENTANMELDUNG

(21) Anmeldenummer: **90104655.7**

(22) Anmeldetag: **12.03.90**

(51) Int. Cl.$^5$: **A61K 31/50, A61K 31/135, A61K 31/16, A61K 31/275, A61K 31/22, A61K 31/40, A61K 31/535**

(30) Priorität: **16.03.89 DE 3908531**

(43) Veröffentlichungstag der Anmeldung:
**19.09.90 Patentblatt 90/38**

(84) Benannte Vertragsstaaten:
**AT BE CH DE DK ES FR GB GR IT LI LU NL SE**

(71) Anmelder: **Dr. Karl Thomae GmbH**
**Postfach 1755**
**D-7950 Biberach 1(DE)**

(72) Erfinder: **Mauz, Annerose, Dipl.-Biol., Dr.**
**Habsburgerstrasse 59**
**D-7945 Langenenslingen-Emerfeld(DE)**

Erfinder: **Diederen, Willi, Dr., Pharmakologe**
**Haldenstrasse 1a**
**D-7950 Biberach 1(DE)**
Erfinder: **van Meel, Jacques, Dr.,**
**Pharmakologe**
**Schubertweg 4**
**D-7951 Mittelbiberach(DE)**
Erfinder: **Wienen, Wolfgang, Dr.,Dipl.-Biol.**
**Am Schiessberg 28**
**D-7951 Aepfingen(DE)**
Erfinder: **Austel, Volkhard, Dr., Dipl.-Chem.**
**Kapellenweg 16**
**D-7950 Biberach 1(DE)**

(54) **Arzneimittel mit einer positiv inotropen Wirkung, enthaltend eine synergistisch wirkende Mischung, bestehend aus einem Benzimidazol und einem beta-Blocker, deren Herstellung und deren Verwendung.**

㊼ Arzneimittel mit einer positiv inotropen Wirkung, enthaltend ein Benzimidazol der Formel

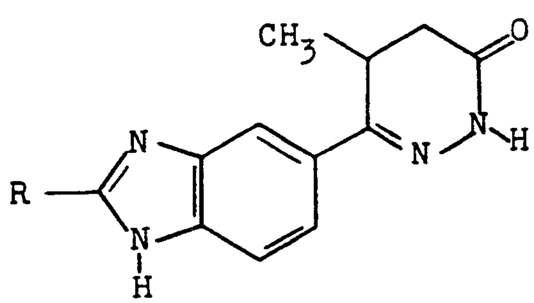

,(I)

in der
R eine Alkyl-, Hydroxyphenyl- oder Methoxyphenylgruppe bedeutet, und einen $\beta$-Blocker neben einem oder mehreren inerten Trägerstoffen.

EP 0 387 762 A2

# Arzneimittel mit einer positiv inotropen Wirkung, enthaltend eine synergistisch wirkende Mischung, bestehend aus einem Benzimidazol und einem β-Blocker, deren Herstellung und deren Verwendung

In der EP-B-0.008.391 werden u.a. in 2-Stellung substituierte 5-(5-Methyl-3-oxo-4,5-dihydro-2H-6-pyridazinyl)-benzimidazole der Formel

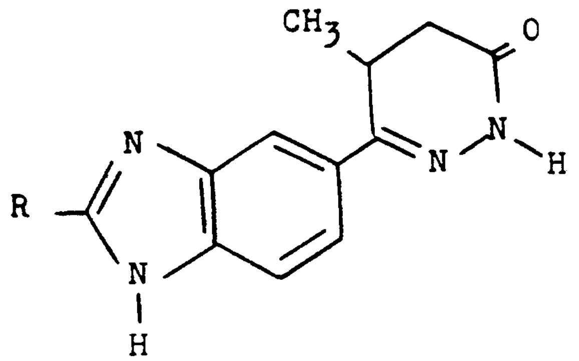

,(I)

in der
R eine Alkylgruppe mit 1 bis 5 Kohlenstoffatomen, eine Hydroxyphenyl oder Methoxyphenylgruppe bedeutet, deren 3H-Tautomere, deren optisch aktive Antipoden und deren physiologisch verträgliche Säureadditionssalze beschrieben, welche wertvolle pharmakologische Eigenschaften aufweisen, insbesondere neben einer antiviralen, Interferon-induzierenden und Ulcus-hemmenden Wirkung eine cardiovasculäre Wirkung, nämlich eine cardiotonische, blutdrucksenkende und/oder antithrombotische Wirkung.

Unter dem Begriff "cardiovasculäre Wirkungen" ist definitionsgemäß eine Wirkung betreffend das Herz und die Gefäße zu verstehen, die im vorliegenden Falle durch eine antithrombotische, cardiotonische und eine Wirkung auf den Blutdruck dokumentiert wird.

Aufgrund dieser pharmakologischen Eigenschaften eignen sich die Verbindungen der EP-B-0.008.391, deren 3H-Tautomere und deren optisch aktive Antipoden sowie deren physiologisch verträgliche Säureadditionssalze mit anorganischen oder organischen Säuren zur Behandlung der chronischen Herzinsuffizienz oder der Angina Pectoris und/oder zur Prophylaxe arterieller Thromboembolien und arteriellen Verschlußkrankheiten, sowie zur Behandlung von Ulcera und zur Bekämpfung von Viren und viralen Erkrankungen.

Deren Verwendbarkeit als Therapeutikum bei der chronischen Herzinsuffizienz beruht also auf deren cardiotonischer Wirkung sowie bei arteriellen Thromboembolien und Verschlußkrankheiten auf deren antithrombotischer Wirkung, insbesondere deren Thrombozytenwirkung.

Außerdem wird in der nichtvorveröffentlichten EP-A-0.330.052 die Kombination bestehend aus einem Benzimidazol der vorstehend erwähnten Formel I und einem β-Blocker, welche antiischämische Wirkungen am Herzen aufweist, beschrieben.

Desweiteren ist bekannt, daß β-Blocker bisher vornehmlich zur Behandlung von ischämischen Herzkrankheiten eingesetzt wurden, bei denen der Herzmuskel noch nicht erkrankt ist, wobei jedoch in letzter Zeit auch versucht wird, die β-Blocker auch bei der chronischen Herzinsuffizienz einzusetzen, um das Herz zu entlasten und seine Pumpfunktion zu verbessern. Bei bestimmten Krankheitsbildern, die mit einer Verminderung der Kontraktionskraft des Herzens einhergehen, z.B. einer durch verschiedene Faktoren bedingten Herzin suffizienz (siehe The American Journal of Cardiology 55, 9A-14A (1985)), zeigen jedoch β-Blocker sowohl im Tierexperiment als auch beim klinischen Einsatz unerwünschte negativ inotrope Wirkungen.

Es wurde nun gefunden, daß die vorstehend erwähnten Benzimidazole der Formel I in Kombination mit β-Blockern die negativ inotropen Wirkungen letzterer nicht nur aufheben, sondern sogar zu einer Verbesserung der Herzfunktion bei körperlicher Belastung führen. Dieser Befund ist überraschend. Denn bei körperlicher Belastung kommt es zu einer physiologischen Steigerung der Herzfunktion über eine Erhöhung der Sympathikus-Aktivität. Diese erhöhte Sympathikus-Aktivität kommt unter β-Blockade nicht mehr zum Tragen. Eine positiv inotrope Substanz kann unter Ruhebedingungen die negativ inotrope Wirkung eines β-Blockers kompensieren. Es ist jedoch nicht vorherzusehen, daß die reflektorische Anpassung der Herzfunktion an körperliche Belastung unter β-Blockade durch eine positiv inotrope Substanz wiederhergestellt wird.

Gegenstand der vorliegenden Erfindung sind somit neue synergistisch wirkende Arzneimittel, welche ein Benzimidazol der obigen allgemeinen Formel I, dessen 3H-Tautomeren, dessen optisch aktiven

Antipoden oder dessen physiologisch verträgliche Säureadditionssalze zusammen mit einem β-Blocker enthalten, deren Herstellung und Verwendung sowie die Verwendung eines derartigen synergistisch wirkenden Gemisches zur Herstellung eines Arzneimittels mit einer positiv inotropen Wirkung.

Bevorzugt sind hierbei diejenigen Benzimidazole der Formel I, in der
R eine Methyl-, 2-Pentyl-, 4-Methoxyphenyl- oder 4-Hydroxyphenylgruppe darstellt,
insbesondere diejenigen Benzimidazole der Formel I, in der
R eine 4-Methoxyphenyl- oder 4-Hydroxyphenylgruppe darstellt, deren 3H-Tautomere, deren optisch aktive Antipoden und deren physiologisch verträgliche Säureadditionssalze.

Als β-Blocker kommt beispielsweise Atenolol, Metoprolol, Pindolol, Penbutolol, Propanolol, Carazolol, Alprenolol, Bupranolol, Bisoprolol, Mepindolol, Metipranolol, Betaxolol, Acebutolol, Nadolol, Sotalol, Bunitrolol, Timolol und Oxprenolol in Betracht.

Besonders bevorzugte Arzneimittelkombinationen sind diejenigen, in denen beide Wirksubstanzen vergleichbare Halbwertzeiten aufweisen, vorzugsweise eine Kombination bestehend aus 2-(4-Methoxyphenyl)-5-(5-methyl-3-oxo-4,5-dihydro-2H-6-pyridazinyl)-benzimidazol (Verbindung A) und einem β-Blocker wie Atenolol, Betaxolol, Metoprolol, Timolol, Nadolol oder Propanolol oder eine Kombination bestehend aus 2-(4-Hydroxyphenyl)-5-(5-methyl-3-oxo-4,5-dihydro-2H-6-pyridazinyl)-benzimidazol (Verbindung B) und einem β-Blocker wie Metoprolol oder Pindolol. Die erstere Kombination eignet sich hierbei insbesondere für die perorale Applikation und die zweite Kombination insbesondere für die intravenöse Applikation.

Die Einzeldosis am Erwachsenen liegt bei einer Verbindung der Formel I zwischen 0,1 und 10,0 mg, vorzugsweise jedoch zwischen 1,0 und 2,5 mg, 1- bis 2-mal täglich, um eine erfindungsgemäße Wirkung zu erzielen.

Demgegenüber kann die Einzeldosis des erfindungsgemäß eingesetzten β-Blockers auf Grund ihrer verschiedenen Wirkungsstärken stark variieren. Diese beträgt beispielsweise pro Tag bei der Verwendung
von Atenolol 25 bis 100 mg, vorzugsweise jedoch 50 mg,
von Metoprolol 50 bis 150 mg, vorzugsweise 100 mg,
von Timolol 5 bis 15 mg, vorzugsweise 5 bis 10 mg,
von Nadolol 25 bis 80 mg, vorzugsweise 30 bis 60 mg, und
von Propanolol 50 bis 100 mg, vorzugsweise 50 mg, verteilt auf eine oder zwei Einzeldosen.

Die erfindungsgemäß neuen Kombinationen bestehend aus einem Benzimidazol der Formel I und einem β-Blocker wurde beispielsweise an der Kombination Verbindung A/Atenolol wie folgt geprüft:

Beeinflussung der reflektorischen Anpassung der Herzfunktion an körperliche Belastung

Hunde (mischrassig, beiderlei Geschlechts, Körpergewicht 22-32 kg) werden mit einer Kombination von 0,2 ml/kg Polamivet $^{(R)}$ und 0,05 ml/kg Combelen $^{(R)}$ prämediziert. Nach endotrachealer Intubation werden die Tiere mit einem Gemisch von 1% Halothan, 24% $O_2$ und 75% $N_2O$ beatmet. Unter sterilen Bedingungen wird der Thorax im linken 5. Intercostalraum eröffnet und ein Konigsberg-Druckaufnehmer in den linken Ventrikel durch eine Stab-Incision am Apex eingeführt. Der Druckaufnehmer wird zur Messung des linksventrikulären Druckes fixiert, das Kabel nach außen geführt und der Thorax verschlossen. Nach der Operation erhalten die Tiere eine 2-monatige Ruhepause, in welcher sie an das Laufband gewöhnt werden.

Versuchsprotokoll:

Durch Gehen auf einem Laufband (Geschwindigkeit des Laufbandes: 0-2-4-6-8 km/h) werden die Hunde einer steigenden körperlichen Belastung ausgesetzt, wobei jede Belastungsstufe 3 Minuten dauert. Ein erster Lauf dient zur Erfassung der Kontrolwerte, der zweite Lauf wird nach i.v. Applikation der Substanzen durchgeführt. Während des gesamten Versuches wird der linksventrikuläre Druck gemessen und Herzfrequenz als auch LV-dP/$dt_{max}$ (ein Maß der Kontraktionskraft des Herzens) erfasst. Die nachfolgende Tabelle enthält die gefundenen Werte:

| Substanz | Dosis i.v. mg/kg | $LV\text{-}dP/dt_{max}$ (mmHg/s) | |
|---|---|---|---|
| | | in Ruhe | bei maximaler Belastung im Versuch (8 km/h) |
| Kontrolle | --- | 1,88 ± 0,13 | 2,98 ± 0,20 |
| Verbindung A | 0,3 | 3,40 ± 0,18 | 4,77 ± 0,18 |
| Kontrolle | --- | 1,98 ± 0,20 | 2,88 ± 0,19 |
| Atenolol | 0,1 | 1,88 ± 0,16 | 2,15 ± 0,17 |
| Kontrolle | --- | 1,95 ± 0,15 | 3,28 ± 0,21 |
| A/Atenolol | 0,3/0,1 | 2,57 ± 0,14 | 4,18 ± 0,20 |

Die erfindungsgemäß einsetzten Verbindungen sind gut verträglich. So weist beispielsweise die Verbindung A folgende akute Toxizitäten auf:

| Substanz | $LD_{50}$ | |
|---|---|---|
| | i.v. mg/kg | p.o. mg/kg |
| A | 72 (Ratte) | 600 (Maus) |
| B | >100 (Ratte) | |

Die Toxizitäten der in den Kombinationen erfindungsgemäß verwendeten β-Blocker sind literaturbekannt und in therapeutischen Dosen gut verträglich.

Die erfindungsgemäß eingesetzten Kombinationen sind ebenfalls gut verträglich, so konnte beispielsweise bei den applizierten Dosen der Kombinationen Verbindung A/Atenolol keine toxischen Nebenwirkungen beobachtet werden.

Zur pharmazeutischen Anwendung lassen sich hierzu die vorstehend genannten Wirkstoffe zusammen mit einem oder mehreren inerten üblichen Trägerstoffen und/oder Verdünnungsmitteln, z.B. mit Maisstärke, Milchzucker, Rohrzucker, mikrokristalliner Cellulose, Magnesiumstearat, Polyvinylpyrrolidon, Zitronensäure, Weinsäure, Wasser, Wasser/Ethanol, Wasser/Glyzerin, Wasser/Sorbit, Wasser/Polyethylenglykol, Propylenglykol, Cetylstearilalkohol, Carboxymethylcellulose oder fetthaltige Substanzen wie Hartfett, in übliche galenische Zubereitungen wie Tabletten, Dragées, Kapseln, Pulver, Suspensionen, Tropfen, Ampullen, Säfte oder Zäpfchen einarbeiten.

Die nachfolgende Beispiele sollen die Erfindung näher erläutern:

Beispiel 1

Tabletten mit 1,0 mg 2-(4-Methoxyphenyl)-5-(5-methyl-3-oxo-4,5-dihydro-2H-6-pyridazinyl)-benzimidazol und 50,0 mg Atenolol

| Zusammensetzung: | |
|---|---|
| (01) Wirksubstanz | 1,00 mg |
| (02) Atenolol | 50,00 mg |
| (03) Milchzucker | 47,00 mg |
| (04) Maisstärke | 70,00 mg |
| (05) Polyvinylpyrrolidon | 8,00 mg |
| (06) Aerosil | 3,00 mg |
| (07) Magnesiumstearat | 1,00 mg |
| | 180,00 mg |

Herstellung:

Die Stoffe (01) - (04) werden gemischt, mit einer Lösung von (05) in Ethanol granuliert, getrocknet, gesiebt, mit (06) + (07) gemischt und zu Tabletten mit 8 mm Durchmesser verpreßt.

Beispiel 2

Tabletten mit 0,5 mg 2-(4-Methoxyphenyl)-5-(5-methyl-3-oxo-4,5-dihydro-2H-6-pyridazinyl)-benzimidazol und 50,0 mg Atenolol

| Zusammensetzung: | |
|---|---|
| (01) Wirksubstanz | 0,50 mg |
| (02) Atenolol | 50,00 mg |
| (03) Milchzucker | 47,50 mg |
| (04) Maisstärke | 70,00 mg |
| (05) Polyvinylpyrrolidon | 8,00 mg |
| (06) Aerosil | 3,00 mg |
| (07) Magnesiumstearat | 1,00 mg |
| | 180,00 mg |

Herstellung:

Die Stoffe (01) - (04) werden gemischt, mit einer Lösung von (05) in Ethanol granuliert, getrocknet, gesiebt, mit (06) + (07) gemischt und zu Tabletten mit 8 mm Durchmesser verpreßt.

Beispiel 3

Tabletten mit 1,0 mg 2-(4-Methoxyphenyl)-5-(5-methyl-3-oxo-4,5-dihydro-2H-6-pyridazinyl)-benzimidazol und 30,0 mg Nadolol

| Zusammensetzung: | |
|---|---|
| (01) Wirksubstanz | 1,00 mg |
| (02) Nadolol | 30,00 mg |
| (03) Milchzucker | 67,00 mg |
| (04) Maisstärke | 70,00 mg |
| (05) Polyvinylpyrrolidon | 8,00 mg |
| (06) Aerosil | 3,00 mg |
| (07) Magnesiumstearat | 1,00 mg |
| | 180,00 mg |

Herstellung:

Die Stoffe (01) - (04) werden gemischt, mit einer Lösung von (05) in Ethanol granuliert, getrocknet, gesiebt, mit (06) + (07) gemischt und zu Tabletten mit 8 mm Durchmesser verpreßt.

Beispiel 4

Tabletten mit 1,0 mg 2-(4-Methoxyphenyl)-5-(5-methyl-3-oxo-4,5-dihydro-2H-6-pyridazinyl)-benzimidazol und 60,0 mg Nadolol

| Zusammensetzung: | |
|---|---|
| (01) Wirksubstanz | 1,00 mg |
| (02) Nadolol | 60,00 mg |
| (03) Milchzucker | 37,00 mg |
| (04) Maisstärke | 70,00 mg |
| (05) Polyvinylpyrrolidon | 8,00 mg |
| (06) Aerosil | 3,00 mg |
| (07) Magnesiumstearat | 1,00 mg |
| | 180,00 mg |

Herstellung:

Die Stoffe (01) - (04) werden gemischt, mit einer Lösung von (05) in Ethanol granuliert, getrocknet, gesiebt, mit (06) + (07) gemischt und zu Tabletten mit 8 mm Durchmesser verpreßt.

Beispiel 5

Kapseln mit 1,0 mg 2-(4-Methoxyphenyl)-5-(5-methyl-3-oxo-4,5-dihydro-2H-6-pyridazinyl)-benzimidazol und 50,0 mg Propanolol Retard

| Zusammensetzung: | |
|---|---|
| In einer Schmelze (90° C) aus | |
| 19,00 mg<br>110,00 mg<br>50,00 mg<br>1,00 mg<br>180,00 mg | Carnaubawachs und<br>Stearylalkohol werden<br>Propanolol und<br>Wirksubstanz dispergiert |

Herstellung:

Die Dispersion wird in einem geeigneten Gerät versprüht. Die Düse ist so zu wählen, daß Tröpfchen von 300 - 800 µm Durchmesser entstehen. Die Tröpfchen werden im freien Fall gegen einen auf ca. 5 - 8° C abgekühlten Luftstrom bewegt, so daß sie erstarren. Das so erhaltene Sprühgut wird mit 1 mg Mag nesiumstearat gemischt und in Hartgelatine-Kapseln der Größe 3 abgefüllt.

Beispiel 6

Kapseln mit 1,0 mg 2-(4-Methoxyphenyl)-5-(5-methyl-3-oxo-4,5-dihydro-2H-6-pyridazinyl)-benzimidazol und 100,0 mg Metoprolol Retard

| Zusammensetzung: | |
|---|---|
| In einer Schmelze (90° C) aus | |
| 20,00 mg<br>59,00 mg<br>100,00 mg<br>1,00 mg<br>180,00 mg | Carnaubawachs und<br>Stearylalkohol werden<br>Metoprolol und<br>Wirksubstanz dispergiert |

Herstellung:

Die Dispersion wird in einem geeigneten Gerät versprüht. Die Düse ist so zu wählen, daß Tröpfchen von 300 - 800 µm Durchmesser entstehen. Die Tröpfchen werden im freien Fall gegen einen auf ca. 5 - 8° C abgekühlten Luftstrom bewegt, so daß sie erstarren. Das so erhaltene Sprühgut wird mit 1 mg Magnesiumstearat gemischt und in Hartgelatine-Kapseln der Größe 3 abgefüllt.

Beispiel 7

Tabletten mit 1,0 mg 2-(4-Methoxyphenyl)-5-(5-methyl-3-oxo-4,5-dihydro-2H-6-pyridazinyl)-benzimidazol und 10,0 mg Timolol

| Zusammensetzung: | |
|---|---|
| (01) Wirksubstanz | 1,00 mg |
| (02) Timolol | 10,00 mg |
| (03) Milchzucker | 51,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 120,00 mg |

Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

## Beispiel 8

Tabletten mit 1,0 mg 2-(4-Methoxyphenyl)-5-(5-methyl-3-oxo-4,5-dihydro-2H-6-pyridazinyl)-benzimidazol und 10,0 mg Beta-xolol

| Zusammensetzung: | |
|---|---|
| (01) Wirksubstanz | 1,00 mg |
| (02) Betaxolol | 10,00 mg |
| (03) Milchzucker | 51,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 120,00 mg |

Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

## Beispiel 9

Tabletten mit 1,0 mg 2-(4-Methoxyphenyl)-5-(5-methyl-3-oxo-4,5-dihydro-2H-6-pyridazinyl)-benzimidazol und 100,0 mg Metoprolol

| Zusammensetzung: | |
|---|---|
| (01) Wirksubstanz | 1,00 mg |
| (02) Metoprolol | 100,00 mg |
| (03) Milchzucker | 27,00 mg |
| (04) Maisstärke | 80,00 mg |
| (05) Polyvinylpyrrolidon | 8,00 mg |
| (06) Aerosil | 3,00 mg |
| (07) Magnesiumstearat | 1,00 mg |
| | 220,00 mg |

Herstellung:

Die Stoffe (01) - (04) werden gemischt, mit einer Lösung von (05) in Ethanol granuliert, getrocknet, gesiebt, mit (06) + (07) gemischt und zu Tabletten mit 9 mm Durchmesser verpreßt.

Beispiel 10

Tabletten mit 1,0 mg 2-(4-Methoxyphenyl)-5-(5-methyl-3-oxo-4,5-dihydro-2H-6-pyridazinyl)-benzimidazol und 5,0 mg Timolol

| Zusammensetzung: | |
|---|---|
| (01) Wirksubstanz I | 1,00 mg |
| (02) Timolol | 5,00 mg |
| (03) Milchzucker | 56,00 mg |
| (04) Maisstärke | 50,00 mg |
| (05) Polyvinylpyrrolidon | 5,00 mg |
| (06) Aerosil | 2,00 mg |
| (07) Magnesiumstearat | 1,00 mg |
| | 120,00 mg |

Herstellung:

Die Stoffe (01) - (04) werden gemischt, mit einer Lösung von (05) in Ethanol granuliert, getrocknet, gesiebt, mit (06) + (07) gemischt und zu Tabletten mit 7 mm Durchmesser verpreßt.

Beispiel 11

Ampullen mit 1,0 mg 2-(4-Hydroxyphenyl)-5-(5-methyl-3-oxo-4,5-dihydro-2H-6-pyridazinyl)-benzimidazol-hydrochlorid und 5 mg Metoprolol

| Zusammensetzung: | |
|---|---|
| (01) Wirksubstanz | 1,00 mg |
| (02) Metoprolol | 5,00 mg |
| (03) Mannit | 100,00 mg |
| (04) 1N HCl ad pH 2,7 ca. | 2,4 µl |
| (05) Wasser f. Inj.zwecke ad | 2,0 ml |

Herstellung:

(01) + (02) + (03) werden in Wasser gelöst, mit der Salzsäure wird auf pH 2,7 eingestellt, nach Sterilfiltration in 2 ml Ampullen abgefüllt. Sterilisieren 20 Minuten bei 120° C.

Beispiel 12

Ampullen mit 1,0 mg 2-(4-Hydroxyphenyl)-5-(5-methyl-3-oxo-4,5-dihydro-2H-6-pyridazinyl)-benzimidazol-hydrochlorid und 0,4 mg Pindolol

| Zusammensetzung: | |
|---|---|
| (01) Wirksubstanz | 1,00 mg |
| (02) Pindolol | 0,40 mg |
| (03) Mannit | 100,00 mg |
| (04) 1N HCl ad pH 2,7 ca. | 2,2 µl |
| (05) Wasser f. Inj.zwecke ad | 2,0 ml |

Herstellung:

(01) + (02) + (03) werden in Wasser gelöst, mit der Salzsäure wird auf pH 2,7 eingestellt, nach Sterilfiltration in 2 ml Ampullen abgefüllt. Sterilisieren 20 Minuten bei 120° C.

## Ansprüche

1. Arzneimittel mit einer positiv inotropen Wirkung auf Basis einer Mischung bestehend aus einem Benzimidazol der Formel

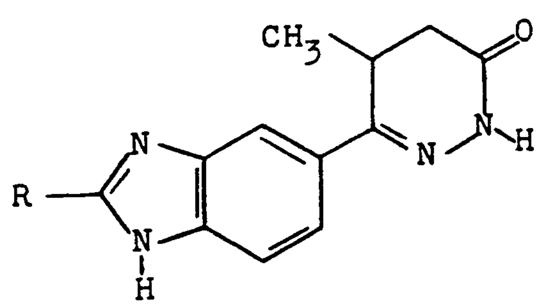

,(I)

in der
R eine Alkylgruppe mit 1 bis 5 Kohlenstoffatomen, eine Hydroxyphenyl- oder Methoxyphenylgruppe bedeutet, dessen 3H-Tautomerem, dessen optisch aktiven Antipoden oder dessen physiologisch verträgli-

chen Säureadditionssalzen und einem $\beta$-Blocker neben Trägerstoffen und/oder anderen Zuschlagstoffen, dadurch gekennzeichnet, daß es ein Benzimidazol der obigen Formel und einen $\beta$-Blocker in einer eine synergistische Wirkung erzeugender Menge enthält.

2. Arzneimittel gemäß Anspruch 1, dadurch gekennzeichnet, daß dieses ein Benzimidazol der Formel I, in der R eine Methyl-, 2-Pentyl-, 4-Methoxyphenyl- oder 4-Hydroxyphenylgruppe darstellt, enthält.

3. Arzneimittel gemäß Anspruch 1, dadurch gekennzeichnet, daß dieses ein Benzimidazol der Formel I, in der R eine 4-Methoxyphenyl- oder 4-Hydroxyphenylgruppe darstellt, enthält.

4. Arzneimittel gemäß Anspruch 1, dadurch gekennzeichnet, daß dieses ein Benzimidazol der Formel I, in der R eine 4-Methoxyphenyl- oder 4-Hydroxyphenylgruppe darstellt, enthält.

5. Arzneimittel gemäß den Ansprüchen 1 bis 4, dadurch gekennzeichnet, daß dieses als Einzeldosis 0,1 bis 10 mg, vorzugsweise jedoch 1 bis 2,5 mg, eines Benzimidazols der Formel I gemäß den Ansprüchen 1 bis 4 enthält.

6. Arzneimittel gemäß den Ansprüchen 1 bis 5, dadurch gekennzeichnet, daß dieses als $\beta$-Blocker Atenolol, Metoprolol, Pindolol, Penbutolol, Propanolol, Carazolol, Alprenolol, Bupranolol, Bisoprolol, Mepindolol, Metipranolol, Betaxolol, Acebutolol, Nadolol, Sotalol, Bunitrolol, Timolol und Oxprenolol enthält.

7. Arzneimittel gemäß den Ansprüchen 4 und 5, dadurch gekennzeichnet, daß dieses als $\beta$-Blocker Atenolol, Betaxolol, Metoprolol, Timolol, Nadolol oder Propanolol enthält.

8. Arzneimittel gemäß den Ansprüchen 4 und 5, dadurch gekennzeichnet, daß dieses als $\beta$-Blocker Metoprolol oder Pindolol enthält.

9. Verwendung eines Gemisches bestehend aus einem Benzimidazol gemäß den Ansprüchen 1 bis 4 und einem $\beta$-Blocker zur Herstellung eines Arzneimittels mit einer positiv inotropen Wirkung.

10. Verfahren zur Herstellung eines Arzneimittels gemäß den Ansprüchen 1 bis 8, dadurch gekennzeichnet, daß eine Verbindung gemäß den Ansprüchen 1 bis 4 in Kombination mit einem $\beta$-Blocker in einen oder mehrere inerte Trägerstoffe und/oder Verdünnungsmittel eingearbeitet wird.